# EUROPÄISCHE PATENTSCHRIFT

(11) **EP 0 710 096 B1**
(45) Veröffentlichungstag und Bekanntmachung des Hinweises auf die Patenterteilung: **13.08.1997**
(21) Anmeldenummer: 94921643.6
(22) Anmeldetag: 12.07.1994
(51) Int. Cl.: A61F 13/15

(54) **SAUGKÖRPER, INSBESONDERE FÜR WINDELN, DAMENBINDEN, SLIPEINLAGEN UND DERGL. UND VERFAHREN ZU DESSEN HERSTELLUNG**
ABSORBENT BODY, IN PARTICULAR FOR DIAPERS, SANITARY TOWELS, PANTIES PROTECTIONS AND THE LIKE, AND PROCESS FOR PRODUCING THE SAME
CORPS ABSORBANT, NOTAMMENT POUR COUCHES, SERVIETTES HYGIENIQUES, PROTEGE-SLIPS ET ANALOGUES, ET SON PROCEDE DE FABRICATION

(30) Priorität: 23.07.1993 DE 4324802
(43) Veröffentlichungstag der Anmeldung: 08.05.1996
(73) Patentinhaber: Paul Hartmann Aktiengesellschaft, D-89522 Heidenheim (DE)
(72) Erfinder: MALOWANIEC, Krzysztof, D., D-89522 Heidenheim (DE)
(74) Vertreter: Becker, Maria, Dipl.-Phys.
(86) Internationale Anmeldenummer: EP9402281
(87) Internationale Veröffentlichungsnummer: WO9503020

(56) Entgegenhaltungen:
- EP-A- 0 021 662
- EP-A- 0 160 572
- WO-A-93/11727
- AU-B- 512 002
- DE-A- 3 137 839
- DE-A- 3 205 931
- DE-A- 3 716 271
- GB-A- 2 124 907
- US-A- 4 333 463

## Beschreibung

Die Erfindung betrifft einen Saugkörper wie er im Oberbegriff des Anspruchs 1 angegeben ist sowie ein Verfahren zu dessen Herstellung.

Derartige Saugkörper werden in hygienischen Artikeln, insbesondere Einmalartikeln, wie Windeln, Höschenwindeln, Damenbinden, Slipeinlagen und dergleichen eingesetzt. Dabei ist es wichtig, daß die aufzunehmende Körperflüssigkeit vom Saugkörper schnell aufgenommen, verteilt und gespeichert wird, so daß der Saugkörper bei einem erneuten Anfall von Körperflüssigkeit diese wiederum schnell aufnehmen kann.

Insbesondere bei Saugkörpern, die für Windeln gedacht sind, ist eine schnelle Flüssigkeitsaufnahme, -verteilung und -speicherung erforderlich, da hier große Mengen von Körperflüssigkeit auf einmal anfallen.

Es ist bereits ein aus einer Fasermasse (Fluff) gebildeter Saugkörper insbesondere für Windeln bekannt (AU-B-512 002), der aus zwei Saugkörperschichten besteht. Die eine Schicht weist dabei runde, sich durch die Schicht hindurcherstreckende Öffnungen auf, während die andere Schicht durchgehend ist. Wenn die beiden aufeinanderliegenden Schichten in geeigneter Weise zusammengepreßt werden, bilden sich an den Stellen der Öffnungen Speicherbereiche für aufzunehmende Flüssigkeit mit geringerer Dichte, während an den Stellen der zwischen den Öffnungen gelegenen Stege Bereiche mit größerer Dichte entstehen, die aufgrund ihrer dochtartigen Saugwirkung zur Verteilung von Körperflüssigkeit im Saugkörper dienen.

Dieser bekannte Saugkörper hat den Nachteil, daß von ihm aufzunehmende Flüssigkeit nur relativ langsam aufgesaugt und gespeichert werden kann.

Aus der EP-A 0 160 572 ist ein Saugkörper bekannt, der eine zweischichtige Struktur aufweist, deren eine Schicht Durchbrechungen aufweist und dazu dient, die anfallende Flüssigkeit zu absorbieren und zu halten, während die andere Schicht die angefallene Flüssigkeit innerhalb des Saugkörpers verteilt.

Der Erfindung liegt die Aufgabe zugrunde, einen verbesserten Saugkörper der eingangs genannten Art sowie ein Verfahren zu dessen Herstellung bereitzustellen.

Erfindungsgemäß wird diese Aufgabe durch den Gegenstand des Anspruchs 1 bzw. das Verfahren nach Anspruch 12 gelöst.

Durch die erfindungsgemäß unterschiedliche Ausbildung von Saug- und Basisschicht wird eine Funktionstrennung im Saugkörper erreicht. Die Saugschicht mit ihrer geringeren Dichte und dem geringeren Retentionswert der Fasern nimmt infolge ihrer relativ lockeren und offenporigen Struktur anfallende Flüssigkeit sehr schnell auf und leitet sie an die Basisschicht weiter, die infolge ihrer größeren Dichte und dem höheren Retentionswert der Fasern als Flüssigkeitsspeicher dient. Dabei wird durch die hineinragende Struktur der Saugschicht in die Basisschicht eine deutlich höhere Kontakt- und Flüssigkeitsübergabefläche erzielt.

Die vergrößerte Kontaktfläche zwischen Saugschicht und Basisschicht ermöglicht einen schnelleren Übertritt der Flüssigkeit von der Saugschicht in die Basisschicht und gewährleistet eine zuverlässige Verbindung dieser beiden Schichten.

Besonders vorteilhaft ist es dabei, daß für die Herstellung des erfindungsgemäßen Saugkörpers vorgefertigte Materialien verwendet werden können, wodurch bei vereinfachter Fertigung eine gleichbleibende Qualität der Saugkörper erreicht wird.

Durch die geringere Dichte der Saugschicht im Bereich der Durchbrechungen gelangt die von der Saugschicht aufgenommene Flüssigkeit gezielt in den Bereich der Durchbrechungen der Basisschicht.

Zweckmäßige Formen der Durchbrechungen der Basisschicht sind in den Ansprüchen 2 und 3 angegeben.

Der zweckmäßige Anteil der offenen Flächen der Durchbrechungen an der Gesamtfläche der Basisschicht ist im Anspruch 4 angegeben. Besonders bevorzugt ist es dabei, wenn die offenen Flächen etwa 10 % der Gesamtfläche ausmachen, also etwa im Bereich zwischen 8 % oder 9 % bis 12 oder 14 % liegen. Hierbei läßt sich eine gewünschte Vergrößerung der wirksamen Kontaktfläche für anfallende Körperflüssigkeit erreichen, ohne daß die Speicherkapazität der Basisschicht wesentlich beeinträchtigt wird.

Die unterschiedlichen Retentionswerte der Fasermaterialien für die beiden Schichten lassen sich bei den Weiterbildungen nach Anspruch 5 oder 6 besonders einfach einstellen.

Eine Verbesserung der Speicherkapazität bzw. der Naßfestigkeit der Schichten wird durch die Weiterbildungen der Erfindung nach Anspruch 7 bis 9 erreicht.

Die Aufnahmekapazität des Saugkörpers läßt sich gemäß Anspruch 10 weiter vergrößern, wobei zweckmäßigerweise entsprechend Anspruch 11 der Anteil der Quellstaffsubstanzen in der Basisschicht stets größer gehalten wird als in der Saugschicht, um das Verhalten bei der Flüssigkoitsverteilung in der Saugschicht nicht zu beeinträchtigen.

In besonders vorteilhafter Weise läßt sich der erfindungsgemäße Saugkörper mit einem Verfahren nach den Ansprüchen 12 bis 19 herstellen.

Dabei ist es besonders vorteilhaft, daß das Fasermaterial für die Saugschicht beim Ablegen auf die Basisschicht in die Durchbrechungen hineingesaugt wird, so daß sich eine einheitliche Saugschicht mit davon vorstehenden Teilen bildet, die sich in die Durchbrechungen hineinerstrecken. Hierdurch wird eine gute Fixierung der Saugschicht auf der Basisschicht erreicht. Durch die bevorzugte Verringerung der Dichte des Fasermaterials der Saugschicht im Bereich der Durchbrechungen und insbesondere in den Durchbrechungen wird die gewünschte verbesserte Flüssigkeitsverteilung und -übergabe von der Saugschicht an die Basisschicht erreicht

Die Erfindung wird im folgenden beispielsweise anhand der schematischen Zeichnung näher erläutert. In dieser zeigt:
- Figur 1a und 1b: jeweils eine Draufsicht auf eine Basisschicht des Saugkörpers mit verschieden ausgestalteten Durchbrechungen,
- Figur 2: einen Schnitt durch einen Saugkörper im wesentlichen nach Linie II-II in Figur 1b und
- Figur 3: eine vereinfachte Darstellung einer Anlage zur Herstellung des Saugkörpers.

In den verschiedenen Figuren der Zeichnung sind einander entsprechende Teile mit gleichen Bezugszeichen versehen.

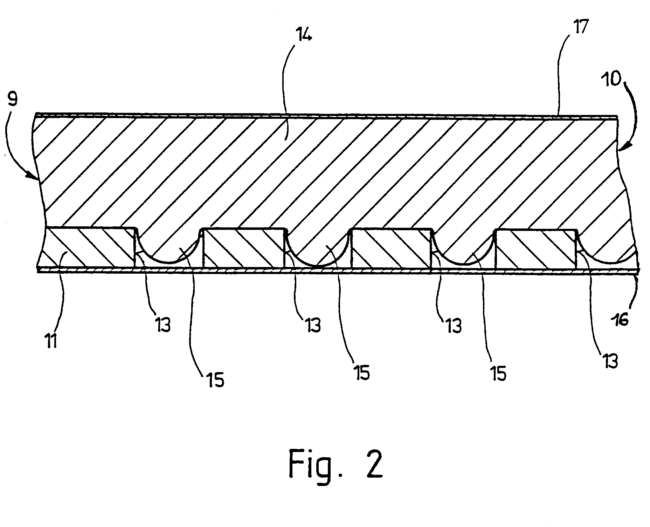

Figur 2 zeigt einen zweiteiligen Saugkörper 10, der eine Basisschicht 11 aus hydrophilen Fasern und ein daraufliegendes Saugkissen 9 aufweist. Die Basisschicht 11 enthält vorzugsweise superabsorbierende Quellstoffsubstanzen, die mit dem Fasermaterial vermischt sind. Außerdem kann bei Bedarf Bindemittel dem Material der Basisschicht 11 beigemischt sein.

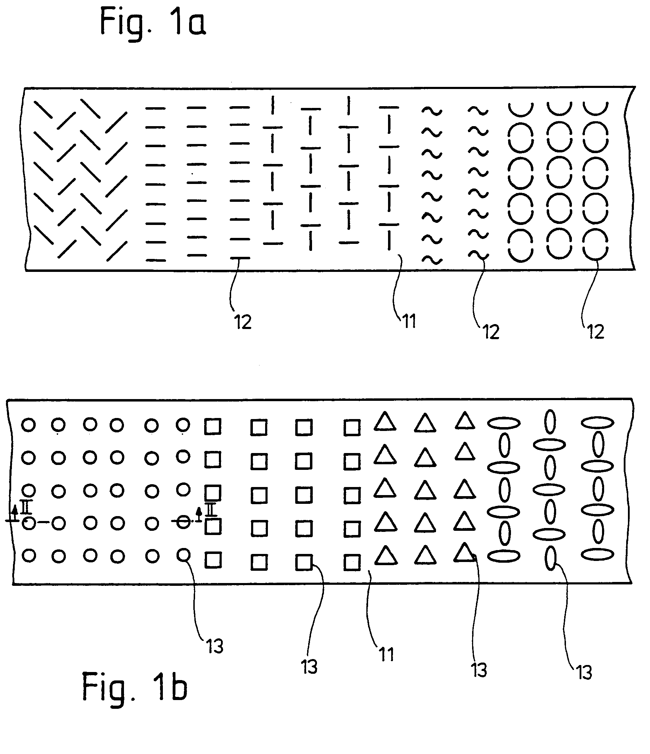

Wie Figuren 1a und 1b zeigen, weist die Basisschicht 11 eine Vielzahl von Durchbrechungen auf, die als gerade, gewellte oder gekrümmte Schlitze 12 oder als Löcher 13 mit unterschiedlicher geometrischer Gestalt ausgebildet sein können. Die Schlitze 12 und Löcher 13 können dabei in verschiedenen regelmäßigen oder unregelmäßigen Mustern über die Basisschicht 11 verteilt angeordnet sein. Auch die Größe der Schlitze 12 und Löcher 13 kann dabei innerhalb eines Musters variieren.

Die offenen Flächen der Durchbrechungen machen dabei zwischen 0 % (bei den Schlitzen 12) und 50 % der Gesamtfläche der Basisschicht 11 aus. Vorzugsweise sind 5 bis 25 % der Gesamtfläche von den Löchern 13 eingenommene offene Flächen, wobei insbesondere ein Anteil von etwa 10 %, also ein Anteil zwischen 8 und 14 % an der Gesamtfläche für die offenen Flächen der Löcher 13 zu wählen ist, um die Oberfläche der Basisschicht 11 zu vergrößern, ohne die Flüssigkeitsaufnahmekapazität so zu verringern, daß sich die Basisschicht 11 nicht mehr als Speicherschicht für einen Saugkörper 10 eignet.

Das auf die Basisschicht 11 aufgebrachte Saugkissen 9 des Saugkörpers 10 weist eine Saugschicht 14 auf, die mit aus deren Fasermaterial bestehenden, einzelnen Schichtvorsprüngen, wie Noppen 15, versehen ist. Diese Schichtvorsprünge sind mit den Schlitzen 12 oder Löchern 13 der Basisschicht 11 in Eingriff.

Die Basisschicht 11 ist auf der von der Saugschicht 14 abgewandten Seite mit einer Wäscheschutzfolie 16 versehen, während auf der entsprechenden Außenseite der Saugschicht 14 eine poröse Abdeckschicht 17 aufgebracht ist, die beispielsweise aus Vliesstoff bestehen kann.

Zur Herstellung des beschriebenen Saugkörpers 10 wird zunächst die Basisschicht 11 als Bahn 11' aus hydrophilen Fasern vorgefertigt. Die Bahn 11' kann dann z. B. auf eine Vorratstrommel 20 aufgewickelt sein oder unmittelbar der folgenden Verarbeitung zugeführt werden.

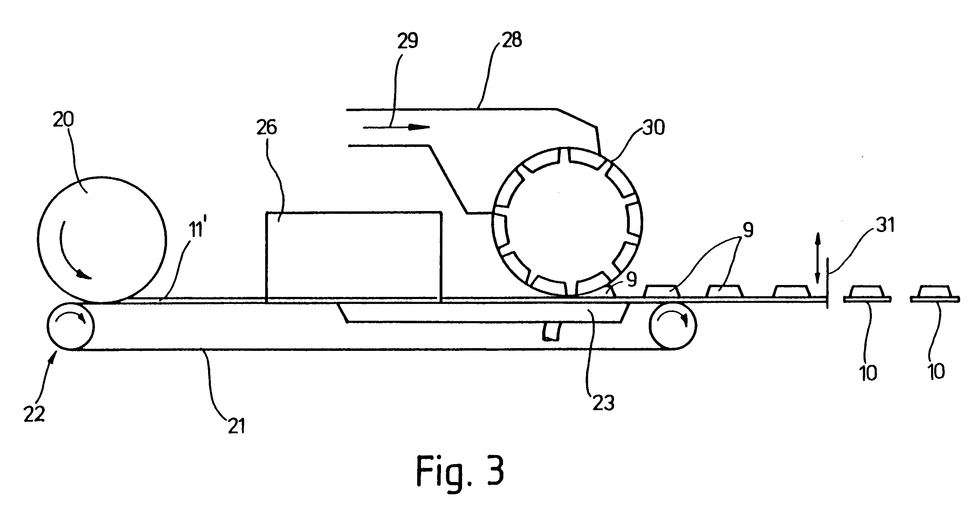

Die die Basisschicht 11 bildende Bahn 11' wird also z. B., wie in Fig. 3 gezeigt, von einer Vorratstrommel 20 abgewickelt und dann von einem Siebband 21 einer Transporteinrichtung 22 einer Stanz- oder Perforiervorrichtung 26 zugeführt, in der entweder die Durchbrechungen als bloße Einschnitte oder Schlitze 12 durch Einschneiden oder aber als runde, rechteckige, dreieckige oder ovale Löcher 13 durch Ausstanzen gebildet werden.

Die vorgefertigte und mit Durchbrechungen versehene Bahn 11' wird dann über eine mit einer nicht dargestellten Unterdruckquelle verbundenen Saugvorrichtung 23 geführt.

Im Bereich der Saugvorrichtung 23 werden vorzugsweise vorgefertigte Saugkissen 9 auf der perforierten Bahn 11' abgelegt.

Zur Bildung der vorgefertigten Saugkissen 9 werden hydrophile Fasern durch einen Schacht 28 mittels eines Luftstroms 29 einer geeigneten Siebtrommel 30 zugeführt, deren Inneres in bekannter Weise mit einer Unterdruckquelle verbunden ist. Den hydrophilen Fasern können dabei je nach Bedarf Bindemittel und/oder superabsorbierende Quellstoffsubstanzen beigemischt sein.

Für die Saugkissen 9 werden Fasern verwendet, die im Vergleich mit den Fasern für die Basisschicht eine verringerte Wasseraufnahmefähigkeit gemessen als Retentionswert der Fasern besitzen. Eine Einzelfaser der Saugschicht kann also im Vergleich mit einer Einzelfaser der Basisschicht weniger Flüssigkeit aufnehmen und halten.

Die Saugkissen 9 bzw. deren Saugschicht 14 wird von dem unterhalb der Bahn 11' mittels der Saugvorrichtung 23 erzeugten Unterdruck auf die Bahn 11' angesaugt, wobei Teile des Fasermaterials des Saugkissens 9 in die Durchbrechungen 12, 13 der Bahn 11' eindringen, so daß sich in den Durchbrechungen 12, 13 von der Saugschicht 14 vorstehende Teile 15 bilden.

Hierdurch wird erreicht, daß das Saugkissen 9 fest auf der Basisschicht 11 fixiert wird.

Durch das Einsaugen der von der Saugschicht 14 vorstehenden Schichtvorsprünge 15 in die Schlitze 12 oder Löcher 13, also in die Durchbrechungen der Basisschicht 11 wird das hydrophile Fasermaterial des Saugkissens 9 in den Durchbrechungen 12, 13 und in deren Bereichen gegenüber den restlichen Teilen der Saugschicht 14 aufgelockert, so daß die Schichtvorsprünge 15 und die Saugschicht 14 in deren Bereich eine verringerte Dichte aufweisen. Es treten dadurch Dichteunterschiede auf, die den Transport von von der Saugschicht 14 aufgenommener Flüssigkeit zur Basisschicht 11 erleichtern.

Außerdem bewirkt die beschriebene Befestigung des Saugkissens 9 auf der Basisschicht 11, daß die Kontakflächen zwischen dem Saugkissen 9 und der Basisschicht 11 und damit die Aufnahmefläche für Flüssigkeit in die Basisschicht 11 vergrößert ist. Somit wird das Einsaugen von Flüssigkeit in die Basisschicht 11 erleichtert und beschleunigt.

Das Saugkissen 9 dient also im wesentlichen zur Flüssigkeitsaufnahme und zu deren Verteilung, während die Basisschicht 11 in erster Linie der Flüssigkeitsspeicherung dient. Es ist daher besonders vorteilhaft, wenn die Basisschicht 11 einen deutlich höheren Anteil an superabsorbierenden Quellstoffsubstanzen enthält als das Saugkissen 9, da so die Flüssigkeitsspeicherfunktion der Basisschicht 11 unterstützt wird, während der relativ geringere Anteil an Quellstoffsubstanzen im Saugkissen 9 die Flüssigkeitsverteilungsfunktion der Saugschicht nicht beeinträchtigt.

Nach dem Aufbringen des Saugkissens 9 auf die Bahn 11 wird die Saugkörperbahn von einer Schneid- oder Trennvorrichtung 31, die nur schematisch angedeutet ist, in einzelne Saugkörper 10 queraufgetrennt, mit einer Wäscheschutzfolie 16 und einer Abdeckschicht 17 versehen und zur weiteren Verarbeitung abtransportiert.

Wird der erfindungsgemäße Saugkörper in einer Windel verwendet, so wird der bei einer Miktion anfallende Urin zunächst sehr schnell von der Saugschicht 14 aufgenommen und über den Saugkörper 10 verteilt, wobei die Bereiche des Saugkörpers 10 mit verringerter Dichte, die im Bereich der Durchbrechungen 12, 13 vorgesehen sind, den anfallenden Urin verstärkt zur Basisschicht 11 ableiten. Durch diese zusätzliche Drainagewirkung und die infolge der Durchbrechungen vergrößerte Oberfläche der Basisschicht 11 wird der Urin von der als Speicherschicht dienenden Basisschicht 11 schneller aufgenommen, so daß der eigentliche Saugkörper 10, der durch die Saugschicht 14 des Saugkissens 9 gebildet wird, schneller für die nächste Miktion, also das nächste Wasserlassen, freigemacht wird.

Die in einer Windel anfallende Körperflüssigkeit fließt also aufgrund der relativ lockeren und offenporigen Struktur des Saugkissens und des geringen Retentionswertes der für das Saugkissen verwendeten Fasern nahezu ungehindert zur Basisschicht, um von dieser aufgenommen und gespeichert zu werden. Auf diese Weise wird die Flüssigkeit sofort vom Saugkörper der Windel aufgenommen, um dann in der Basisschicht gespeichert und zurückgehalten zu werden.

## Patentansprüche

1. Saugkörper, insbesondere für Windeln, Damenbinden, Slipeinlagen und dergleichen, mit einer Basisschicht (11) aus hydrophilen Fasern, die mit Durchbrechungen (12, 13) versehen ist, und mit einer Saugschicht (14) aus hydrophilen Fasern, die mindestens auf einer Seite der Basisschicht (11) auf dieser aufliegt, wobei die Saugschicht (14) mit aus deren Fasermaterial bestehenden Schichtvorsprüngen (15) mit den Durchbrechungen (12, 13) der Basisschicht (11) in Eingriff ist,
und die spezifische Wasseraufnahmefähigkeit der gesamten Struktur der Basisschicht (11) größer ist als die der Saugschicht (14) und
die Saugschicht (14) eine Einheits-Flüssigkeitsmenge schneller aufnimmt als die Bassischicht (11),
**dadurch gekennzeichnet,**
- daß die Dichte der Basisschicht (11) größer als die Dichte der Saugschicht (14) ist,
- daß die Saugschicht (14) im Bereich der Durchorechungen (12, 13) und die sich in die Durchbrechungen hineinerstreckenden Schichtvorsprünge (15) eine kleinere Dichte aufweisen als die restliche Saugschicht (14),
- und daß die Wasseraufnahmefähigkeit, gemessen als Retentionswert, der hydrophilen Fasern der Basisschicht (11) größer ist als die der Fasern in der Saugschicht (14).

2. Saugkörper nach Anspruch 1, dadurch gekennzeichnet, daß die Durchbrechungen der Basisschicht (11) zur Aufnahme der von der Saugschicht (14) abragenden Schichtvorsprünge (15) durch Schlitze (12) gebildet sind.

3. Saugkörper nach Anspruch 1, dadurch gekennzeichnet, daß die Durchbrechungen der Basisschicht (11) zur Aufnahme der von der Saugschicht (14) abragenden Schichtvorsprünge (15) durch ausgestanzte Löcher (13) gebildet sind, die die Form von Kreisen, Dreiecken, Rechtecken oder anderen geometrischen Figuren besitzen.

4. Saugkörper nach einem der vorhergehenden Ansprüche, dadurch gekennzeichnet, daß die offenen Flächen der Durchbrechungen (12, 13) zwischen 0 % und 50 %, vorzugsweise zwischen 5 % und 25 %, insbesondere 10 % der Gesamtfläche der Basisschicht (11) ausmachen.

5. Saugkörper nach einem der vorhergehenden Ansprüche, dadurch gekennzeichnet, daß das Fasermaterial für die Saugschicht (14) zur Verringerung der Wasseraufnahmefähigkeit, gemessen als Retentionswert der hydrophilen Fasern vorbehandelt ist.

6. Saugkörper nach Anspruch 1 bis 4, dadurch gekennzeichnet, daß das Fasermaterial für die Saugschicht (14) von synthetischen Fasern aus Polyester, Polypropylen oder dergleichen gebildet ist.

7. Saugkörper nach einem der vorhergehenden Ansprüche, dadurch gekennzeichnet, daß die aus hydrophilen Fasern gebildete Basisschicht (11) superabsorhierende Quellstoffsubstanzen enthält.

8. Saugkörper nach einem der vorhergehenden Ansprüche, dadurch gekennzeichnet, daß die aus hydrophilen Fasern gebildete Basisschicht (11) Bindemittel enthält.

9. Saugkörper nach einem der vorhergehenden Ansprüche, dadurch gekennzeichnet, daß die Saugschicht (14) Bindemittel enthält.

10. Saugkörper nach einem der vorhergehenden Ansprüche, dadurch gekennzeichnet, daß die aus hydrophilen Fasern gebildete Saugschicht (14) und die davon vorstehenden Teile (15) superabsorbierende Quellstoffsubstanzen enthalten.

11. Saugkörper nach Anspruch 7 bis 10, dadurch gekennzeichnet, daß der Anteil der Quellstoffsubstanzen in der Basisschicht (11) bezogen auf das Gesamtgewicht des Basisschichtmaterials größer ist als der Anteil der Quellstoffsubstanzen in der Saugschicht (14) bezogen auf das Gesamtgewicht des Saugschichtmaterials.

12. Verfahren zur Herstellung eines Saugkörpers für Windeln, Damenbinden, Slipeinlagen und dergleichen, insbesondere nach einem der vorhergehenden Ansprüche, bei dem eine Bahn (11') aus hydrophilen Fasern vorgefertigt wird, bei dem die vorgefertigte Bann (11') mit Durchbrechungen (12, 12', 13) versehen wird, bei dem auf die vorgefertigte Bahn (11') eine weitere Schicht aus hydrophilen Fasern als Saugschicht (14) aufgebracht wird, bei dem die Bahn (11') mit der Saugschicht (14) in einzelne Saugkörper durch Trennen aufgeteilt wird, bei dem zum Aufbringen der Saugschicht (14) auf eine Seite der Bahn (11') Fasermaterial abgelegt wird und bei dem auf der anderen Seite der Bahn (11') ein Unterdruck erzeugt wird, so daß Teile des Fasermaterials der Saugschicht (14) in die Durchbrechungen (12, 13) der Bahn gesaugt werden,
**dadurch gekennzeichnet,**
daß das Fasermaterial der Saugschicht (14) im Bereich der Durchbrechungen (12, 13) und der in den Durchbrechungen befindlichen Teile (15) gegenüber dem Fasermaterial der restlichen Saugschicht (14) schwächer verdichtet wird.

13. Verfahren nach Anspruch 12, dadurch gekennzeichnet, daß die Fasermaterial dem Saugschicht aufnehmenden Durchbrechungen in die Bann (11') eingeschnitten werden, wobei Schlitze (12) entstehen.

14. Verfahren nach Anspruch 12, dadurch gekennzeichnet, daß die Fasermaterial der Saugschicht aufnehmenden Durchbrechungen in Form von Kreisen, Rechtecken, Dreiecken oder anderen geometrischen Figuren ausgestanzt werden, wobei entsprechende Löcher (13) entstehen.

15. Verfahren nach einem der Ansprüche 12 bis 14, dadurch gekennzeichnet, daß dem hydrophilen Fasermaterial zum Vorfertigen der Bahn (11') superabsorbierende Quellstoffsubstanzen beigemischt werden.

16. Verfahren nach einem der Ansprüche 12 bis 15, dadurch gekennzeichnet, daß dem hydrophilen Fasermaterial zum Vorfertigen der Bahn (11') Bindemittel beigemischt wird.

17. Verfahren nach einem der Ansprüche 12 bis 16, dadurch gekennzeichnet, daß dem hydrophilen Fasermaterial für die Saugschicht (14) superabsorbierende Quellstoffsubstanzen beigemischt werden.

18. Verfahren nach einem der Ansprüche 12 bis 17, dadurch gekennzeichnet, daß dem hydrophilen Fasermaterial für die Saugschicht (14) Bindemittel beigemischt werden.

19. Verfahren nach Anspruch 17 oder 18, dadurch gekennzeichnet, daß das Beimischen der Quellstoffsubstanzen und/oder Bindemittel zum Fasermaterial für die Saugschicht (14) vor dem Ablegen des Fasermaterials auf die die Basisschicht (11) bildende Bahn (11') erfolgt.

## Claims

1. Absorbent body, especially for diapers, sanitary towels, panties protections and the like, comprising a basic layer (11) made from hydrophilic fibres and provided with openings (12, 13), and an absorbent layer (14) made from hydrophilic fibres and overlying the basic layer (11) by at least one of its sides, where the absorbent layer (14) engages the openings (12, 13) of the basic layer (11) by projections (15) that extends from the absorbent layer and consists of the same fibre material as the latter,
and where the specific water absorption capacity of the entire structure of the basic layer (11) is greater than that of the absorbent layer (14) and
the absorbent layer (14) is capable of absorbing a quantity unit of liquid more rapidly than the basic layer (11),
**characterised in that**
- the density of the basic layer (11) is greater than the density of the absorbent layer (14),
- the absorbent layer (14), in the area of the openings (12, 13), and the projections (15) extending from the layer into the openings have a lower density than the remaining absorbent layer (14),
- and the water absorption capacity, defined as retention value, is higher with the hydrophilic fibres of the basic layer (11) than with the fibres of the absorbent layer (14).

2. Absorbent body according to claim 1, characterised in that the openings in the basic layer (11) intended for receiving the projections (15) extending from the absorbent layer (14) are formed by slots (12).

3. Absorbent body according to claim 1, characterised in that the openings in the basic layer (11) intended for receiving the projections (15) extending from the absorbent layer (14) are formed by punched-out holes (13) exhibiting the shape of circles, triangles, rectangles or other geometric shapes.

4. Absorbent body according to any of the preceding claims, characterised in that the open areas of the openings (12, 13) account for between 0 % and 50 %, preferably between 5 % and 25 %, especially 10 % of the total area of the basic layer (11).

5. Absorbent body according to any of the preceding claims, characterised in that the fibre material of the absorbent layer (14) is pre-treated with a view to reducing the water absorption capacity, defined as retention value, of the hydrophilic fibres.

6. Absorbent body according to claims 1 to 4, characterised in that the fibre material for the absorbent layer (14) consists of synthetic fibres made from polyester, polypropylene or the like.

7. Absorbent body according to any of the preceding claims, characterised in that the hydrophilic fibre basic layer (11) contains superabsorbent swelling substances.

8. Absorbent body according to any of the preceding claims, characterised in that the hydrophilic fibre basic layer (11) contains binding agents.

9. Absorbent body according to any of the preceding claims, characterised in that the absorbent layer (14) contains binding agents.

10. Absorbent body according to any of the preceding claims, characterised in that the hydrophilic fibre absorbent layer (14), and the projections (15) extending therefrom, contain superabsorbent swelling substances.

11. Absorbent body according to claims 7 to 10, characterised in that the proportion of swelling substances contained in the basic layer (11), related to the total weight of the material of the basic layer, is higher than the proportion of swelling substances contained in the absorbent layer (14), related to the total weight of the material of the absorbent layer.

12. Process for producing an absorbent body for diapers, sanitary towels, panties protections and the like, especially according to any of the preceding claims, comprising the steps of prefabricating a web (11') consisting of hydrophilic fibres, providing the prefabricated web (11') with openings (12, 12', 13), applying an additional layer of hydrophilic fibres as an absorbent layer (14) onto the prefabricated web (11'), dividing the web (11') with the absorbent layer (14) applied thereon, into separate absorbent bodies by a cutting process, depositing fibre material on one side of the web (11') for the purpose of applying the absorbent layer (14), and generating a vacuum on the other side of the web (11') for drawing portions of the fibre material of the absorbent layer 14 into the openings (12, 13) of the web,
**characterised in that**
the fibre material of the absorbent layer (14), in the area of the openings (12, 13), and the portions (5) present in the openings, are compressed to a lesser degree than the fibre material of the rest of the absorbent layer (14).

13. Process according to claim 12, characterised in that the openings intended to accommodate the fibre material of the absorbent body are cut into the web (11') so as to form slots (12).

14. Process according to claim 12, characterised in that the openings intended to accommodate the fibre material of the absorbent body are punched out from the web in the form of circles, rectangles, triangles or other geometric shapes to produce corresponding holes (13).

15. Process according to any of claims 12 to 14, characterised in that for prefabricating the web (11'), superabsorbent swelling substances are admixed to the hydrophilic fibre material.

16. Process according to any of claims 12 to 15, characterised in that for prefabricating the web (11'), binding agents are admixed to the hydrophilic fibre material.

17. Process according to any of claims 12 to 16, characterised in that superabsorbent swelling substances are admixed to the hydrophilic fibre material for the absorbent layer (14).

18. Process according to any of claims 12 to 16, characterised in that binding agents are admixed to the hydrophilic fibre material for the absorbent layer (14).

19. Process according to claim 17 or 18, characterised in that the step of admixing the swelling substances and/or the binding agents to the fibre material for the absorbent layer (14) is carried out before the step of depositing the fibre material on the web (11') forming the basic layer (11).

## Revendications

1. Corps absorbant, notamment pour couches, serviettes hygiéniques, protègeslips et analogues; avec une couche de base (11) en fibres hydrophiles munie d'évidements (12, 13) et d'une couche absorbante (14) en fibres hydrophiles reposant au moins d'un côté de la couche de base (11) sur cette dernière, la couche absorbante (14) étant avec ses saillies (15) en matériau fibreux en contact avec les évidements (12, 13) de la couche de base (11), et la propriété hygroscopique spécifique de toute la structure de la couche de base (11) étant plus élevée que celle de la couche absorbante (14) et la couche absorbante (14) absorbant une quantité de liquide unitaire plus rapidement que la couche de base (11),
**caractérisé en ce que**
- la densité de la couche de base (11) est plus grande que la densité de la couche absorbante (14),
- la couche absorbante (14) présente dans la zone des évidements (12, 13) et les saillies (15) de couche s'étendant dans les évidements une densité plus petite que le reste de la couche absorbante (14),
- et que la propriété hygroscopique, mesurée comme valeur de rétention, des fibres hydrophiles de la couche de base (11) est plus élevée que celle des fibres dans la couche absorbante (14).

2. Corps absorbant selon la revendication 1, caractérisé en ce que les évidements de la couche de base (11) recevant les saillies (15) sortant de la couche absorbante (14) sont formées par des fentes (12).

3. Corps absorbant selon la revendication 1, caractérisé en ce que les évidements de la couche de base (11) recevant les saillies (15) sortant de la couche absorbante (14) sont formés par des trous poinçonnés (13) en forme de cercles, triangles, rectangles ou autres figures géométriques.

4. Corps absorbant selon l'une des revendications précédentes, caractérisé en ce que les surfaces ouvertes des évidements (12, 13) présentent entre 0 % et 50 %, de préférence entre 5 % et 25 %, notamment 10 % de la surface totale de la couche de base (11).

5. Corps absorbant selon l'une des revendications précédentes, caractérisé en ce que le matériau fibreux pour la couche absorbante (14) est prétraité pour réduire la propriété hygroscopique, mesurée comme valeur de rétention.

6. Corps absorbant selon l'une des revendications 1 à 4, caractérisé en ce que le matériau fibreux pour la couche absorbante (14) est formé de fibres synthétiques en polyester, polypropylène ou analogues.

7. Corps absorbant selon l'une des revendications précédentes, caractérisé en ce que la couche de base (11) formée de fibres hydrophiles contient des substances gonflantes superabsorbantes.

8. Corps absorbant selon l'une des revendications précédentes, caractérisé en ce que la couche de base (11) formée de fibres hydrophiles contient des liants.

9. Corps absorbant selon l'une des revendications précédentes, caractérisé en ce que la couche absorbante (14) contient des liants.

10. Corps absorbant selon l'une des revendications précédentes, caractérisé en ce que la couche absorbante (14) formée de fibres hydrophiles et les parties (15) en faisant saillie contient des substances gonflantes superabsorbantes.

11. Corps absorbant selon l'une des revendications 7 à 10, caractérisé en ce que la part des substances gonflantes dans la couche de base (11) rapportée au poids total du matériau de la couche de base est plus grande que la part des substances gonflantes dans la couche absorbante (14) rapportée au poids total du matériau de la couche absorbante.

12. Procédé pour la fabrication d'un corps absorbant pour couches, serviettes hygiéniques, protège-slips et analogues, notamment selon l'une des revendications précédentes, dans lequel un pan (11') en fibres hydrophiles est préfabriqué, dans lequel le pan préfabriqué (11') est muni d'évidements (12, 12', 13), dans lequel une autre couche en fibres hydrophiles est appliquée sur le pan préfabriqué (11') comme couche absorbante (14), dans lequel le pan (11') avec la couche absorbante (14) est partagé par séparation en différents corps absorbants, dans lequel du matériau fibreux est déposé sur un côté du pan (11') pour l'application de la couche absorbante (14) et dans lequel une souspression est générée sur l'autre côté du pan (11') de sorte que des parties du matériau fibreux de la couche absorbante (14) sont aspirées dans les évidements du pan,
**caractérisé en ce que**
le matériau fibreux de la couche absorbante (14) est rendu moins dense par rapport au matériau fibreux du reste de la couche absorbante (14) dans la zone des évidements (12, 13) et des parties (15) se trouvant dans les évidements.

13. Procédé selon la revendication 12, caractérisé en ce que les évidements recevant le matériau fibreux de la couche absorbante sont découpés dans le pan (11'), des fentes (12) en résultant.

14. Procédé selon la revendication 12, caractérisé en ce que les évidements recevant le matériau fibreux de la couche absorbante sont poinçonnés en forme de cercle, rectangles, triangles ou autres figures géométriques, des trous (13) adéquats en résultant.

15. Procédé selon l'une des revendications 12 à 14, caractérisé en ce que des substances gonflantes superabsorbantes sont additionnées au matériau fibreux hydrophile pour la préfabrication du pan (11').

16. Procédé selon l'une des revendications 12 à 15, caractérisé en ce que du liant est additionné au matériau fibreux hydrophile pour la préfabrication du pan (11').

17. Procédé selon l'une des revendications 12 à 16, caractérisé en ce que des substances gonflantes superabsorbantes sont additionnées au matériau fibreux hydrophiles pour la couche absorbante (14).

18. Procédé selon l'une des revendications 12 à 17 caractérisé en ce que du liant est additionné au matériau fibreux hydrophile pour la couche absorbante (14).

19. Procédé selon la revendication 17 ou 18, caractérisé en ce que l'addition des substances gonflantes et/ou des liants au matériau fibreux pour la couche absorbante (14) est effectuée avant l'application du matériau fibreux sur le pan (11') formant la couche de base (11).
